# EUROPÄISCHE PATENTSCHRIFT

(11) **EP 0 455 072 B1**
(45) Veröffentlichungstag und Bekanntmachung des Hinweises auf die Patenterteilung: **30.08.1995**
(21) Anmeldenummer: 91106335.2
(22) Anmeldetag: 19.04.1991
(51) Int. Cl.: A61N 1/05, G01N 27/30

(54) **Silberchlorid-Bezugselektrode**
Silver chloride reference electrode
Electrode de référence au chlorure d'argent

(30) Priorität: 02.05.1990 DE 4014112
(43) Veröffentlichungstag der Anmeldung: 06.11.1991
(73) Patentinhaber: Pacesetter AB, 171 95 Solna (SE)
(72) Erfinder: Preidel, Walter, Dr., W-8520 Erlangen (DE)
(74) Vertreter: Lettström, Richard Wilhelm

(56) Entgegenhaltungen:
- EP-A- 0 170 509
- EP-A- 0 393 188
- WO-A-82/01306
- FR-A- 2 378 529
- GB-A- 2 215 844
- US-A- 4 589 418
- JOURNAL OF THE ELECTROCHEMICAL SOCIETY Band 134, Nr. 1, Januar 1987,Seiten 132-134, Manchester, NH, US; W.R. CIESLAK et al.: "The Fabrication and Performance of a Ag/AgCl Reference Electrode in Thionyl Chloride Electrolyte"

## Beschreibung

Die Erfindung betrifft eine Bezugselektrode mit einer auf einer Silberschicht befindlichen Schicht aus Silberchlorid.

Bezugs- oder Referenzelektroden dienen zum Messen von Potentialen anderer Elektroden, indem sie mit diesen quasi zu einer elektrochemischen Zelle kombiniert werden. Die wichtigsten Bezugselektroden sind die Normalwasserstoffelektrode, die Kalomelelektrode und die Silberchloridelektrode (Ag/AgCl-Elektrode).

Aus der WO 82/01306, welche Blutgassensoren betrifft, ist ein Kohlendioxidsensor bekannt, der eine Sensorelektrode aufweist, die an einen Isolator aus Epoxidharz gebunden ist, sowie eine im Isolator gehaltene ringförmige Silberbezugselektrode, die mit Silberchlorid beschichtet ist. Der Sensor ist mit einer Schicht aus hydrophilem Polyurethan versehen, die sowohl die Sensorelektrode als auch die Silberbezugselektrode bedeckt.

Elektrochemische Sensoren und Herzschrittmacher, die im Körper von Patienten implantiert werden, benötigen implantierbare, langzeitstabile Bezugselektroden. Bei Sensoren werden in diesem Zusammenhang in der Literatur lediglich Ag/AgCl-Elektroden vorgeschlagen. Bei derartigen Bezugselektroden ist jedoch bei der geforderten längeren Implantationsdauer eine Vergiftung des Körpers mit Silber nicht auszuschließen (siehe dazu: "Herzschrittmacher", Bd. 8 (1988), Seiten 149 bis 152, sowie "PACE", Vol. 12 (1989), Seiten 1000 bis 1007).

Aufgabe der Erfindung ist es, eine Bezugselektrode mit einer auf einer Silberschicht befindlichen Schicht aus Silberchlorid derart auszugestalten, daß im Betrieb möglichst wenig Silber abgegeben wird, und deshalb auch bei einer Langzeitimplantation im Körper keine durch Silber verursachten Schädigungen auftreten können.

Dies wird erfindungsgemäß durch eine Bezugselektrode mit den Merkmalen des Patentanspruchs 1 erreicht.

Bei der erfindungsgemäßen Silberchlorid-Bezugselektrode ist - aufgrund des speziellen Aufbaus - gewährleistet, daß eine Diffusion von Silber bzw. Silberionen in den Körper über lange Zeit weitgehend unterbunden und somit unterhalb eines zumutbaren Wertes gehalten wird. Untersuchungen an Versuchstieren haben gezeigt, daß die abgegebenen geringen Mengen an Silber tolerierbar sind, weil sie keine signifikante Erhöhung der Silberkonzentration im Blut ergeben.

Bei der erfindungsgemäßen Bezugselektrode ist die Silberchloridschicht mit einer Schicht aus einer Poly(perfluoralkylen)-sulfonsäure abgedeckt, wobei die Säuregruppen neutralisiert sind; dazu dient im allgemeinen Nafion. Derartige Schichten, die zwischen 1 und 100 »m dick sein können, sind semipermeabel, so daß im Betrieb der erforderliche elektrische Kontakt zwischen der Elektrode und dem umgebenden Elektrolyten gegeben ist.

Für eine Langzeitimplantation ist es vorteilhaft, die Schicht aus Poly(perfluoralkylen)-sulfonsäure mit einem isolierenden Film aus einem körperverträglichen Polymeren zu versehen, wobei der Polymerfilm die Schicht aus der Poly(perfluoralkylen)-sulfonsäure flächenmäßig nicht vollständig abdeckt. Auf diese Weise hat die Bezugselektrode lediglich durch den über die Silberchloridschicht hinausragenden und nicht durch den Polymerfilm abgedeckten Teil der Schicht aus Poly(perfluoralkylen)-sulfonsäure Kontakt zum umgebenden Elektrolyten. Hierbei besteht somit eine Diffusionsbarriere, die - auch auf Dauer - nur von sehr geringen Silbermengen überwunden werden kann. Als Polymere dienen dabei vorzugsweise Epoxidharze, Polyurethane oder Polyorganosiloxane, d.h. Silicone.

Die erfindungsgemäße Bezugselektrode kann bei elektrochemischen Sensoren, insbesondere Sauerstoffsensoren und Glucosesensoren, und auch bei physiologisch gesteuerten Herzschrittmachern Verwendung finden. Sie kann dabei entweder - in planarer Form, d.h. scheibenförmig - auf bzw. in einem (Metall-)Gehäuse eines implantierbaren Gerätes angeordnet sein oder - in Form einer zylindrischen Elektrode, d.h. als rotationssymmetrischer Körper - auf einem implantierbaren Kabel. Bezugselektroden nach der Erfindung können im übrigen mit Dampf sterilisiert werden.

Die Herstellung der vorliegenden Bezugselektrode kann beispielsweise - vereinfacht - in der Weise erfolgen, daß auf eine Silberschicht bzw. eine Hülse aus Silber eine Silberchloridschicht aufgebracht wird; dies kann durch Auftragen von geschmolzenem Silberchlorid erfolgen. Die Silberchloridschicht wird dann mit einer Schicht aus Poly(perfluoralkylen)-sulfonsäure versehen, beispielsweise in Form einer Membran, die - falls (noch) erforderlich - neutralisiert wird. Gegebenenfalls wird auf diese Schicht bzw. Membran noch ein isolierender Film aus einem körperverträglichen Polymeren aufgebracht, was bei einem zylindrischen Aufbau in der Weise erfolgen kann, daß darüber ein (aufgeweiteter) Schlauch aus entsprechendem Material geschoben wird, beispielsweise ein Siliconschlauch.

Vorzugsweise wird die erfindungsgemäße Bezugselektrode gemäß Verfahrensanspruch 4 hergestellt. Dabei wird dir (äußere) Oberfläche einer Silberschicht, die in planarer oder zylindrischer Form vorliegen kann, anodisch in Silberchlorid übergeführt, beispielsweise unter Verwendung einer Kochsalzlösung. Auf die Silberchloridschicht, deren Oberfläche aufgerauht werden kann, wird dann eine Lösung von Poly(perfluoralkylen)-sulfonsäure aufgebracht, beispielsweise in Form einer handelsüblichen Nafionlösung (5 %ige Lösung in Isopropanol). Danach wird das Lösungsmittel entfernt, was beispielsweise durch Trocknen bei einer Temperatur zwischen 70 und 120°C erfolgt. Anschließend wird noch neutralisiert, wozu im allgemeinen eine Kochsalz- oder Ringerlösung dient; hierbei werden die SO₃H-Gruppen im wesentlichen in SO₃Na-Gruppen übergeführt. Die Vollständigkeit des Ionenaustausches kann dabei durch Messung des Potentials der Elektrode - gegen eine andere Ag/AgCl-Bezugselektrode - festgestellt werden: es soll möglichst Null betragen.

Gegebenenfalls wird die Schicht aus ionenleitendem Material noch mit einer Abdeckschicht versehen. Dazu wird auf die Schicht aus der Poly(perfluoralkylen)-sulfonsäure eine Lösung eines filmbildenden, körperverträglichen Polymeren aufgebracht. Dies kann beispielsweise eine Epoxidharzlösung oder eine Lösung von Polyurethan in N-Methylpyrrolidon sein. Anschließend wird dann noch das Lösungsmittel entfernt.

Anhand von zwei Figuren, in denen bevorzugte Ausführungsformen der erfindungsgemäßen Bezugselektrode dargestellt sind, soll die Erfindung noch weiter verdeutlicht werden.

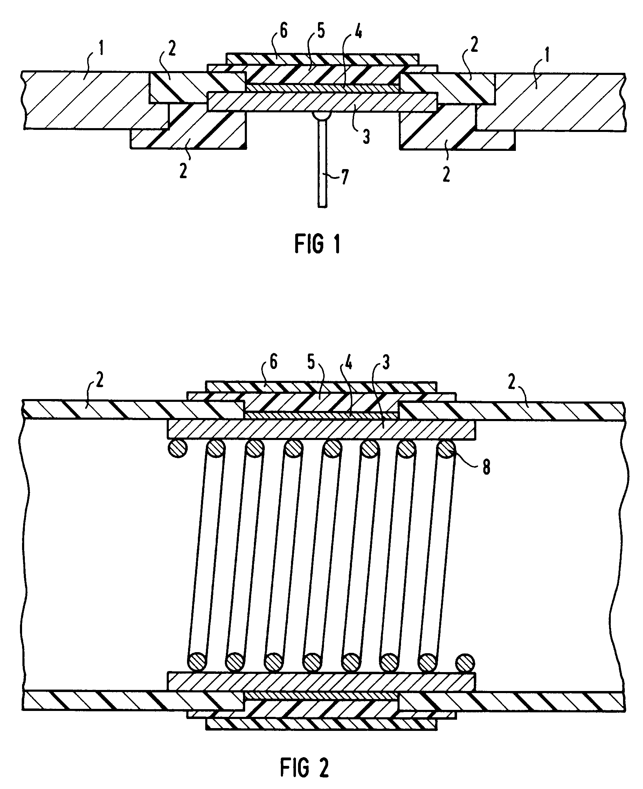

In den Figuren, von denen Fig. 1 eine planare Bezugselektrode und Fig. 2 eine zylindrische Bezugselektrode zeigt, sind gleiche Elemente mit gleichen Bezugsziffern bezeichnet. Dabei bedeutet: 1 Gehäuse, 2 Dichtung bzw. Isolierung, 3 Silberschicht bzw. -hülse, 4 Silberchloridschicht, 5 Schicht aus wasseraufnehmendem, ionenleitendem Material, d.h. Poly(perfluoralkylen)-sulfonsäure, 6 isolierender Film bzw. Schlauch, 7 Metalldraht (zur Kontaktierung), 8 Metallwendel (zur Kontaktierung).

## Patentansprüche

1. Bezugselektrode mit einer auf einer Silberschicht (3) befindlichen Schicht (4) aus Silberchlorid, die mit einer Schicht (5) aus wasseraufnehmendem, ionenleitendem Material versehen ist, welche flächenmäßig über die Silberchloridschicht (4) hinausragt, **dadurch gekennzeichnet,** daß die Schicht (5) aus wasseraufnehmendem, ionenleitendem Material eine Schicht aus Poly(perfluoralkylen)-sulfonsäure ist, wobei die Säuregruppen neutralisiert sind.

2. Bezugselektrode nach Anspruch 1, **dadurch gekennzeichnet,** daß die Schicht (5) aus der Poly(perfluoralkylen)-sulfonsäure mit einem isolierenden Film (6) aus einem körperverträglichen Polymeren versehen ist, wobei der Polymerfilm (6) die Schicht (5) aus der Poly(perfluoralkylen)-sulfonsäure flächenmäßig nicht vollständig abdeckt.

3. Bezugselektrode nach Anspruch 2, **dadurch gekennzeichnet,** daß das Polymere ein Epoxidharz, Polyurethan oder Polyorganosiloxan ist.

4. Verfahren zur Herstellung einer Bezugselektrode nach einem der Ansprüche 1 bis 3, bei dem die Oberfläche einer Silberschicht (3) anodisch in Silberchlorid übergeführt wird, auf die Silberchloridschicht (4) eine Lösung von Poly(perfluoralkylen)-sulfonsäure aufgebracht wird welche flächenmäßig über die Silberchloridschicht (4) hinausragt, das Lösungsmittel entfernt wird, und die Poly(perfluoralkylen)-sulfonsäure neutralisiert wird.

5. Verfahren nach Anspruch 4, **dadurch gekennzeichnet**, daß auf die Schicht (5) aus der Poly(perfluoralkylen)-sulfonsäure eine Lösung eines filmbildenden, körperverträglichen Polymeren aufgebracht wird, und daß das Lösungsmittel entfernt wird.

6. Verfahren nach Anspruch 4 oder 5, **dadurch gekennzeichnet,** daß die Oberfläche der Silberchloridschicht (4) aufgerauht wird.

## Claims

1. Reference electrode having located on a silver layer (3) a layer (4) of silver chloride, which is provided with a layer (5) of water-absorbing, ion-conducting material, which projects beyond the silver chloride layer (4) in terms of surface area, characterised in that the layer (5) of water-absorbing, ion-conducting material is a layer of poly(perfluoroalkylene)-sulphonic acid, in which the acid groups are neutralised.

2. Reference electrode according to claim 1, characterised in that the layer (5) of the poly(perfluoroalkylene)-sulphonic acid is provided with an isolating film (6) of a polymer which is compatible with the body, wherein the polymer film (6) does not completely cover in terms of surface area the layer (5) of the poly(perfluoroalkylene)-sulphonic acid.

3. Reference electrode according to claim 2, characterised in that the polymer is an epoxy resin, polyurethane or polyorganosiloxane.

4. Method for the production of a reference electrode according to one of the claims 1 to 3, wherein the surface of a silver layer (3) is anodically converted into silver chloride, a solution of poly(perfluoroalkylene)-sulphonic acid is deposited onto the silver chloride layer (4), and projects in terms of area beyond the silver chloride layer (4), the solvent is removed and the poly(perfluoroalkylene)-sulphonic acid is neutralised.

5. Method according to claim 4, characterised in that a solution of a film-forming polymer which is compatible with the body is deposited onto the layer (5) of the poly(perfluoroalkylene)-sulphonic acid, and in that the solution medium is removed.

6. Method according to claim 4 or 5, characterised in that the surface of the silver chloride layer (4) is roughened.

## Revendications

1. Electrode de référence comportant une couche de chlorure d'argent (4) se trouvant sur une couche d'argent (3) et munie d'une couche (5) d'une matière absorbant l'eau, conduisant les tons et qui dépasses en surface de la couche de chlorure d'argent (4), caractérisée en ce que la couche (5), en une matière absorbant l'eau et conduisant les ions, est une couche d'acide poly(perfluoralkylène)-sulfonique, les groupes acide étant neutralisés.

2. Electrode de référence selon la revendication 1, caractérisée en ce que la couche d'acide poly(perfluoralkylène)-sulfonique (5) est munie d'un pellicule isolante (6) d'un polymère acceptable par l'organisme, la pellicule de polymère (6) ne recouvrant pas complètement en surface la couche d'acide poly(perfluoralkylène)-sulfonique (5).

3. Electrode de référence selon la revendication 2, caractérisée en ce que le polymère est une résine époxyde, un polyuréthanne ou un polyorganosiloxane.

4. Procédé de fabrication d'une électrode de référence selon l'une des revendications 1 à 3, dans lequel on transforme anodiquement la surface d'une couche d'argent (3) en chlorure d'argent, on applique sur la couche de chlorure d'argent (4) une solution d'acide poly(perfluoralkylène)-sulfonique, laquelle dépasse en surface de la couche de chlorure d'argent (4), on élimine le solvant et on neutralise l'acide poly(perfluoralkylène)-sulfonique.

5. Procédé selon la revendication 4, caractérisé en ce qu'on applique sur la couche (5) de l'acide poly(perfluoralkylène)-sulfonique une solution d'un polymère filmogène, acceptable par l'organisme, et on élimine le solvant.

6. Procédé selon la revendication 4 ou 5, caractérise en ce qu'on rend rugueuse la surface de la couche de chlorure d'argent (4).
